# EUROPEAN PATENT APPLICATION

(11) **EP 1 600 132 A1**
(43) Date of publication of application: **30.11.2005**
(21) Application number: 04405333.8
(22) Date of filing: 28.05.2004
(51) Int. Cl.: A61F 13/56, A61F 13/15

(54) **Disposable absorbent article with adjustable and prefastening tape**

(71) Applicant: Hyga SA, 2543 Lengnau (CH)
(72) Inventor: The designation of the inventor has not yet been filed

(57) **Abstract**

A disposable absorbent article is pant-like prefastened by a pair of adjustable tape fasteners (20) each including a primary fastener portion (21) and a secondary fastener portion (22). The primary fastener portion pant-like prefasten the front side part (6) and the back side part (7) of the absorbent article along the longitudinal side edges of the side parts. Further, the primary fastener portion (21) is manually releasable or tearable to separate the side parts. The secondary fastener portion (22) comprises an attached end (26) and a free end (27) wherein the attached end is permanently attached to the primary fastener portion (21) or to the back side part (7) and the free end is releasably and refastenably fastened to the front side part (6) or the front waist portion (3) of the absorbent article allowing to adjust a conforming fit about a wearer's waist.

## Description

### FIELD OF THE INVENTION

The present invention relates to disposable absorbent articles for absorbing human exudates. In particular, the present invention relates to disposable absorbent articles such as diapers, training pants or incontinence garments which have an adjustable and pant-like prefastening tape fastener.

### BACKGROUND OF THE INVENTION

It is desired that absorbent articles such as diapers, so-called training pants or incontinence garments provide a close, comfortable fit about the wearer and contain body exudates. Moreover, it is desirable that such absorbent articles, after being soiled, can be removed from the wearer in a convenient and clean manner without undesirably soiling the caregiver or surrounding area such as the clothes of the wearer. In certain circumstances, it is also desirable that such absorbent articles are capable of beeing pulled up or down over the hips of the wearer to allow the wearer or caregiver to easily pull the article on and easily remove the article if it has not been soiled. It is further desirable for the wearer or caregiver to be able to adjust the fit of the waist area of the article.

Conventional diapers are not provided in a prefastened condition and have typically included a front waist portion and a back waist portion which are releasably connected about the hips of the wearer during use by conventional fasteners such as hook and loop or adhesive tape fasteners. Conventional fasteners are well known to those skilled in the art and have typically included a pair of releasable tape fasteners, located on the outermost corners of the diaper in the back waist portion of the diaper and a complimentary attachment panel, located on the exterior surface of the outer cover of the diaper in the front waist portion of the diaper. In such a configuration, the diaper has been positioned between the legs of the wearer while the wearer is lying down and the tape fasteners have been releasably fastened to the attachment panel to secure the back waist portion to the front waist portion of the diaper to secure the diaper about the waist of the wearer. Such conventional diapers are easy to fasten about and remove from the wearer after use without undesirably soiling the wearer or caregiver. However, such conventional diapers are not provided in a pant-like, prefastened configuration and, thus, are not configured to be pant-like pulled up or down over the hips of the wearer.

Conventional training pants are provided to be pulled easily up or down over the hips of the wearer and do not require any of the fastening steps of the conventional diapers. For example, such training pants include prefastened elastic side panels that connect the front waist portion to the back waist portion of the absorbent article. This configuration allows the wearer or caregiver to pull on the training pant in the wearer's standing attitude. Further, the training pant type is fitted to the wearers size due to the expansible waist opening portion. However, many of these conventional training pants have not been completely satisfactory. For example, since the size of the relaxed waist opening is limited, it may be difficult for the wearer or caregiver to insert legs smoothly and the wear may give a feel of being narrow and tight. On the other hand, such training pants have not always been able to achieve a close conforming fit to the wearer while still being able to expand enough to be pulled up and down over the hips of the wearer. Often such training pants fit the waist of the wearer loosely, which can undesirably result in leaks. As a result, many of these articles have not contained bodily exudates as effectively as conventional diapers. Further, many training pants lacked any means for checking the article for soiling without removal of the article and often of the wearer's clothing. Likewise, training pants generally lack any means for removing a soiled diaper without having to tear one or more not refastenable elements (generally seams) of the article which can render the article useless if checked for soiling.

Several attempts have been made to provide absorbent articles that allow the wearer or caregiver to choose between conventional diaper and training pant configurations, or combinations thereof, and properly and comfortably fit a large range of wearer sizes.

EP 0 570 980, US 5,370,634, US 5,531,732, WO 95/29657, WO 99/65438, US 6,524,293, WO 02/069867 and EP 1 350 494 are different embodiments of prefastened, stretchable and adjustable absorbent articles with welded front and back side parts, wherein said side parts are in a facing or overlapping configuration and wherein additional fastening means provide an adjustable waist size and the further possibility of reclosure after tearing the side seams for removal or inspection of the article. These embodiments are not completely satisfactory in at least one of the following properties. To pull on this articles the caregiver has to stretch the relaxed waist opening with both hands so that there is no further possibility to help to insert legs easily. The welding of the side parts has to be made particularly strong due to the stretching forces acting directly on the side seams. At the same time, the side seams or an additional line of weakening should be tearable for removal or inspection of the article. The balance between this forces is difficult to achieve reliably, particularly in embodiments where the welding has to extend through three or more layers of material. Further, the side seams of the facing front and back side parts are welded at the outermost edges what results generally in outwardly extending and relatively hard edge portions. This is not only unsightly, but the hard free edge can easily come into contact with the user's skin at the upper and lower edges thereof, causing discomfort. Further, the welding of the side seams of the facing or overlapping side parts reduces the waist opening size. As compensation, wider materials are required what results in higher costs and more waist. Finally, some of these embodiments are not suitable to use as conventional diapers.

US 5,830,206 is pant-like prefastened by a tape fastener, which allows to release and refasten the absorbent article but is not provided to adjust the waist size.

US 5,624,428 has a tape fastener with an adjustable pant-like pull down feature but ist not prefastened and is not convenient to use as conventional diaper.

US 6,454,752 is a pant-like prefastened disposable absorbent article including a pair of fasteners which provide refastenable side seams and at least one waist size adjustment means. Said fasteners engage the opposing front and back side parts in an facing or overlapping manner, what reduces the waist size or needs wider materials respectively, resulting in higher costs and more waist. Further, to use the unfastened article similar to a conventional diaper, two fastener means on each side have to be attached.

### SUMMARY OF THE INVENTION

In response to the difficulties and problems discussed above, new pant-like disposable absorbent articles having adjustable and prefastening tape fasteners and allowing the wearer or caregiver to choose between conventional diaper and pant configurations have been discovered.

In a first aspect, the present invention concerns a prefastened disposable absorbent article which includes a front waist portion, a back waist portion, a crotch portion which extends between and connects said waist portions and two pairs of opposing front and back side parts. The absorbent article is pant-like prefastened by a pair of adjustable tape fasteners each including a primary fastener portion and a secondary fastener portion. The primary fastener portion is attached to the opposing front and back side parts prefastening the absorbent article along the longitudinal side edges of said front and back side parts. Further, said primary fastener portion is manually releasable or tearable to separate said opposing front and back side parts. The secondary fastener portion comprises an attached end and a free end wherein the attached end is permanently attached to the primary fastener portion or to the back side part and the free end is releasably and refastenably fastened to the outer surface of the other of said opposing side parts providing a wide range of waist size and allowing to adjust a conforming fit about a wearer's waist.

This prefastened disposable absorbent article advantageously provides large waist and leg openings which allows the wearer or caregiver to insert legs and pull up over the hips easily. Moreover, once the prefastened absorbent article is positioned on the hips of the wearer, the waist opening size may be adjusted whereby the required folding of the side parts reduces the leg opening size what both conform to the body of the wearer to effectively contain bodily exudates. Additionally, after releasing or tearing the line of weakening of the prefastening primary fastener portion, the disposable absorbent article is advantageously identical to conventional diapers what allows the wearer or caregiver to choose between conventional diaper and pant configurations.

In a second aspect, the present invention concerns a fastener tape for adjustable and pant-like prefastened absorbent articles including a primary fastener portion and a secondary fastener portion. The primary fastener portion includes a manually releasable bonding section or a manually tearable line of weakening formed by point-wise cuts or perforation and the like. The secondary fastener portion includes an attached end and a free end wherein the attached end is permanently connected, attached or laminated to the primary fastener portion. Further, the line of weakening may be visibly marked by printed perforation patterns or printed diagrams illustrating the tearable property of said line of weakening.

This fastener tape advantageously provides a prefastening and releasable application for pant-like configurations and a conventional fastener application for conventional diaper configurations.

In a third aspect, the present invention concerns a method of producing a pant-like prefastened disposable absorbent article comprising the steps of
a) providing a continuous web of interconnected absorbent chassis
b) attaching a pair of laterally opposed tape fasteners to the back or front side parts
c) selectively cutting said continuous web of interconnected absorbent chassis into discrete absorbent articles
d) folding each of said discrete absorbent articles about a fold line extending in a lateral direction through the crotch portion thereby positioning the front waist portion to the back waist portion and the front side parts to the back side parts in a facing relationship
e) prefastening said discrete absorbent articles by folding the laterally outward extending portions of the laterally opposed tape fasteners along the longitudinal side edges of the facing front and back side parts and attaching the folded fastener portion to the outer surface of the front or back side parts respectively.

This method of producing advantageously provides a prefastening step similar to the proved attachment of conventional tape fasteners on conventional diapers.

### BRIEF DESCRIPTION OF THE DRAWINGS

The invention will now be explained in more details with reference to certain non-limiting embodiments thereof and with the aid of the accompanying drawings, in which:
FIG.**1** representatively shows a perspective view of one embodiment of a prefastened disposable absorbent article according to the present invention, wherein the waist opening is ready to be pulled up over the hips of the wearer;
FIG.**2** representatively shows a perspective view of the disposable absorbent article of FIG.1 wherein the secondary fastener portions of the prefastening tapes have been adjusted to the waist of the wearer after the article has been pulled up over the hips of the wearer;
FIG.**3** representatively shows a plan view of a disposable absorbent article according to the present invention in an embodiment with added side panels in an unfastened, stretched and laid flat condition with the surface of the article which contacts the wearer facing the viewer;
FIG.**4** representatively shows a view from above of a disposable absorbent article according to the present invention wherein the prefastening tapes have a tearable line of weakening;
FIG.**5** representatively shows a view from above of a disposable absorbent article according to the present invention wherein both, the prefastening and the adjustable fastener are refastenable hook and loop type fasteners;
FIG.**6** representatively shows a perspective view of a prefastening tape according to the present invention wherein the secondary fastener portion is permanently attached to the primary fastener portion and wherein the prefastening tape has a manually tearable line of weakening and further printed arrows, illustrating the tearable property of the line of weakening;
FIG.**7** representatively shows a perspective view of a prefastening tape according to the present invention wherein the secondary fastener portion is permanently attached to the primary fastener portion and wherein both, the prefastening and the adjustable fasteners are refastenable hook and loop type fasteners.
FIG.**8** representatively shows a perspective view of a prefastening tape according to the present invention wherein the primary fastener and the secondary fastener are portions of the same fastener strip and wherein the primary fastener portion is permanently attached bet- ween the layers of the back side part and releasably bonded to the front side part.
FIG.**9** representatively shows a perspective view of a prefastening tape according to the present invention wherein the primary fastener and the secondary fastener are portions of the same fastener strip and wherein the primary fastener portion is permanently attached to the back side part and releasably and refastenably connected to the front side part by a hook and loop type fastener.
FIG.**10** representatively shows a perspective view of a prefastening tape according to the present invention wherein the secondary fastener portion is permanently attached to the back side part and releasably connected to the primary fastener portion.
FIG.**11** representatively shows a perspective view of a prefastening tape according to the present invention wherein the secondary fastener portion is permanently attached to the back side part and the primary fastener portion has a manually tearable line of weakening and further printed arrows, illustrating the tearable property of the line of weakening.

### DETAILED DESCRIPTION OF THE INVENTION

The present invention concerns a disposable absorbent article with adjustable and pant-like prefastening tape fasteners, and a method of making the same. The prefastened absorbent article is configured to closely conform to the body of the wearer to effectively contain body exudates while remaining capable of being pulled up or down over the hips and buttocks of the wearer. The pant-like, prefastened disposable absorbent article of the present invention can function similar to a conventional training pant when left in the prefastened, pant-like configuration, or it can be unfastened prior to or during use to function like a conventional diaper. Moreover, the prefastening tapes of the disposable absorbent article include waist size adjustment means allowing the wearer or caregiver to adjust the fit of the article once it is positioned on the hips of the wearer.

The prefastened absorbent article of the present invention will be described in terms of a disposable, pant-like diaper article which is adapted to be worn by infants about the lower torso. In particular, the prefastened absorbent article will be described in terms of a disposable absorbent diaper with adjustable and pant-like prefastening tape fasteners. It is understood that the article and method of the present invention are equally adaptable for other types of absorbent articles such as adult incontinent products, training pants, feminine hygiene products, other personal care or health care garments, and the like.

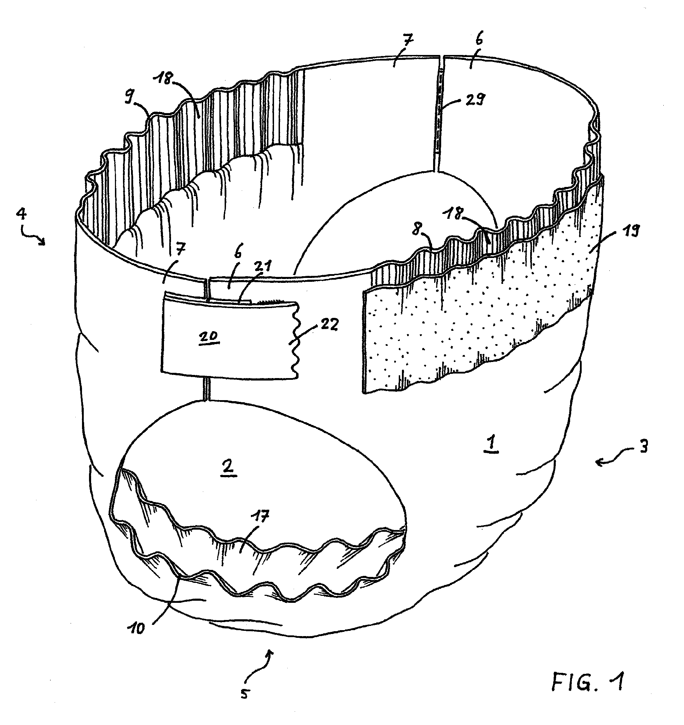

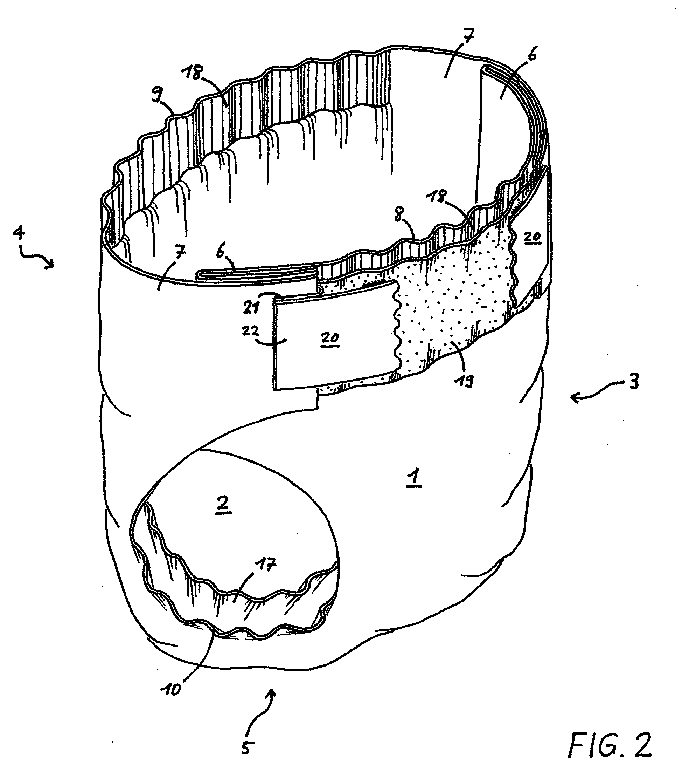

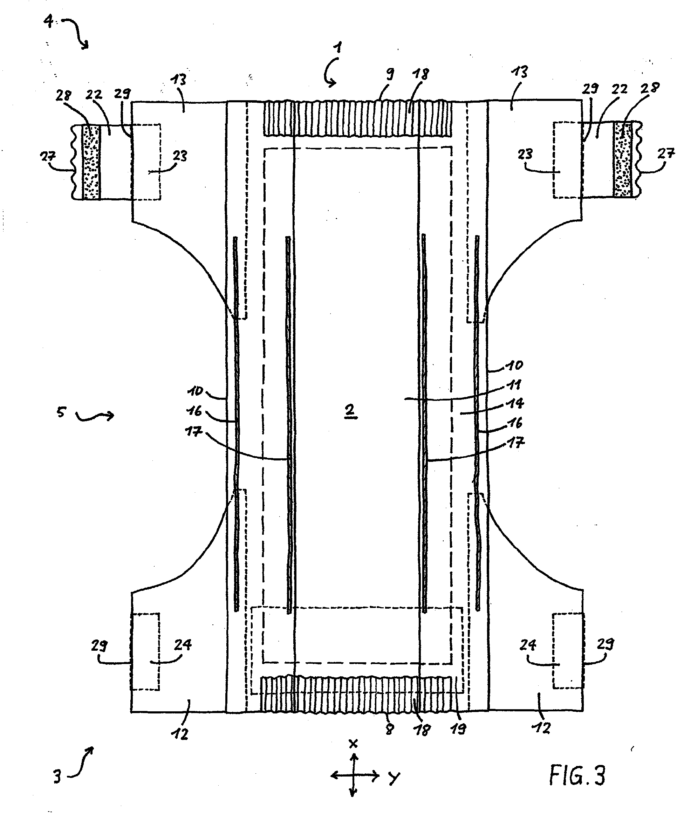

FIG.**1** representatively shows a diaper of the present invention in a ready to be pulled up condition, FIG.**2** representatively shows a diaper of the present invention in an adjusted condition, conforming the waist and the legs of a wearer and FIG.**3** representatively shows a diaper of the present invention in an unfastened, stretched and laid flat condition. The diaper defines a longitudinal direction **x** and a lateral direction **y,** an outer surface **1** and an inner surface **2.** The diaper includes a front waist portion 3, a back waist portion **4,** a crotch portion **5** which extends between and connects the front and back waist portions and two pairs of opposing front side parts **6** and back side parts **7.** Further, the diaper is pant-like prefastened by a pair of adjustable tape fasteners **20.** The opposing front and back side parts **6, 7** and the side edges **10** of the crotch portion generally define leg openings which are adapted to fit about the legs of a wearer in use. As well known to those skilled in the art, the diaper may include leg elastics **16** to provide a snug fit around the leg openings and at least one standing leg gather **17** adjacent the longitudinal side edges of the absorbent core to reduce the likelihood of any leakage therefrom. The front waist edge **8** and the back waist edge **9** generally define the waist opening which is adapted to fit the waist of a wearer in use. The diaper may include elasticated waistband means **18** which are preferably provided at least at one longitudinal end of the absorbent core **11** and serve to provide improved comfort, fit and containment in the article. Such elasticated waistband means are well known in the art. The elastic waistband means **18** are shown in FIG.**3** as lying outside the absorbent core **11,** although it will be understood that they may partially cover the area bounded by the absorbent core. Similarly, the waistband means may extend up to the side edges or side part edges.

The diaper may be of various suitable shapes. For example, the diaper may have an overall rectangular shape, T-shape or an approximately hourglass shape.

The various components of the diaper are integrally assembled together employing various types of suitable attachment means, such as adhesive, thermal and ultrasonic bonds or combinations thereof.

The absorbent core **11** is generally conformable and capable of absorbing and retaining body exudates. The absorbent core may have any of a number of shapes and sizes. For example, the composite absorbent core **11** may be rectangular, T-shape or an approximately hourglass shape. The size and absorbent capacity of the absorbent core should be compatible with the size of the intended wearer and the fluid loading imparted by the intended use of the diaper. The absorbent core **11** comprises various types of wettable, hydrophilic fibrous materials such as cellulosic fibres, synthetic fibres composed of cellulose or cellulose derivatives, particular polyester, polyamide or hydrophilized polypropylene fibres. These fibres or selected blends of the various types of fibres are preferably mixed with particles of a high-absorbency material, commonly known as superabsorbent material, in a homogeneous or layered distribution or in a distribution which varies in the depth direction and/or in the major plane of the core. The absorbent core **11** is positioned between the back sheet **1** defining the outer surface and the top sheet **2** defining the inner surface of the diaper.

The back sheet **1** is preferably impervious to liquids. Materials suitable for these layers are well known and typically the back sheet layer may be formed entirely from or including a polyethylene (PE) film. The back sheet may optionally be composed of a micro-porous "breathable" material which permits vapors to escape from the absorbent core while still preventing liquid exudates from passing through the back sheet. Further, the back sheet **1** may be textile-like formed from a laminate of PE and spunbonded polypropylene (PP) nonwoven material. When the back sheet has this latter construction, it is not necessary for the PE film to have the same extension as the PP nonwoven material, but may be smaller than said sheet, for instance PE film material can be omitted from the side parts **6, 7** of the diaper.

The top sheet **2** is a liquid permeable layer and is comprised, for instance, of nonwoven material compiled from fibres of polyethylene, polypropylene, polyester or mixtures thereof. Viscose fibres may also be used. It is also conceivable to use a perforated plastic layer, for instance a perforated polyethylene layer.

The front and back side parts **6, 7** are well known in suitable constructions and arrangements. FIGS.**1** and **2** representatively show a diaper which is produced in a overall rectangular shape with overall wide extension of the bonded top and back sheet. The front and back side parts **6, 7** remain after cutting the leg opening sections lateral to the absorbent core **11** in the crotch portion of the bonded top and back sheet. FIG.**3** representatively shows a diaper with added front side panels **12** and back side panels **13.** Two front side panels **12** and two back side panels **13** are provided. Each side panel is formed as a single piece in the embodiment shown, although a plurality of pieces laterally or longitudinally in series, or a plurality of layers or panels e.g. in series or partially or fully overlapping may be used to form each side panel. In the preferred case a single piece may be used comprising a plurality of layers, one of which is elastic. The side panels **12, 13** may be each formed with an elastic portion, such as provided by an elastic laminate or elastic threads. The elastic portion may extend so as to cover the whole area of the panels, but preferably should at least cover the area thereof which lies outside the edges of the chassis member **14** up to the laterally outward edge of the panels. Each panel is fixedly attached by adhesive, thermal or ultrasonic bonding to a respective side of the chassis member **14** at attachment locations, preferably between the top sheet **2** and the back sheet **1** as shown. However it should be understood that the side panels **12, 13** may be attached to the internal surface of the top sheet or the external surface of the back sheet if desired, or to both. A still further possibility is that either the back sheet or the top sheet, or a part thereof, may itself be included as part of the side panels and thus form one of the layers of material constituting the side panels. The side panels **12, 13** as representatively illustrated in FIG.**3** can be provided in any suitable shapes which provides the desired fit properties and performance. Further the front side panels and the back side panels can be provided in combinations of the several mentioned constitutions, attachments and shapes. For example, in a preferred embodiment, only the back side panels **13** are formed with an elastic portion. In another aspect the side panels can be provided by an attachment panel superimposed to the outer surface of the back sheet **1** and jutting out the laterally side edges of the chassis member **14.** Such attachment panels are well known as loop material for hook and loop type fastener systems what will be explained below.

The diaper of the different aspects of the present invention includes a pair of fastener tapes **20** which provide a pant-like prefastening and unfastenable primary fastener portion **21** and an adjustable secondary fastener portion **22** for reducing or increasing the waist perimeter dimension after the diaper has been pulled on over the hips of the wearer. In such a configuration, the prefastened diaper can be easily pulled on or off over the legs and hips of the wearer. If the prefastened diaper becomes soiled during use, the primary fastener portion **21** can be unfastened to easily remove the diaper from the waist of the wearer with reduced risk of undesirably soiling the clothes or legs of the wearer. The primary fastener portion can also be easily unfastened to inspect the diaper for possible soiling or before applying the diaper to the wearer if desired. Thus, the diaper is configured to be pulled on or off over the hips of the wearer similar to conventional training pants and can be readily applied or removed like conventional diapers by unfastening the primary fastener portion.

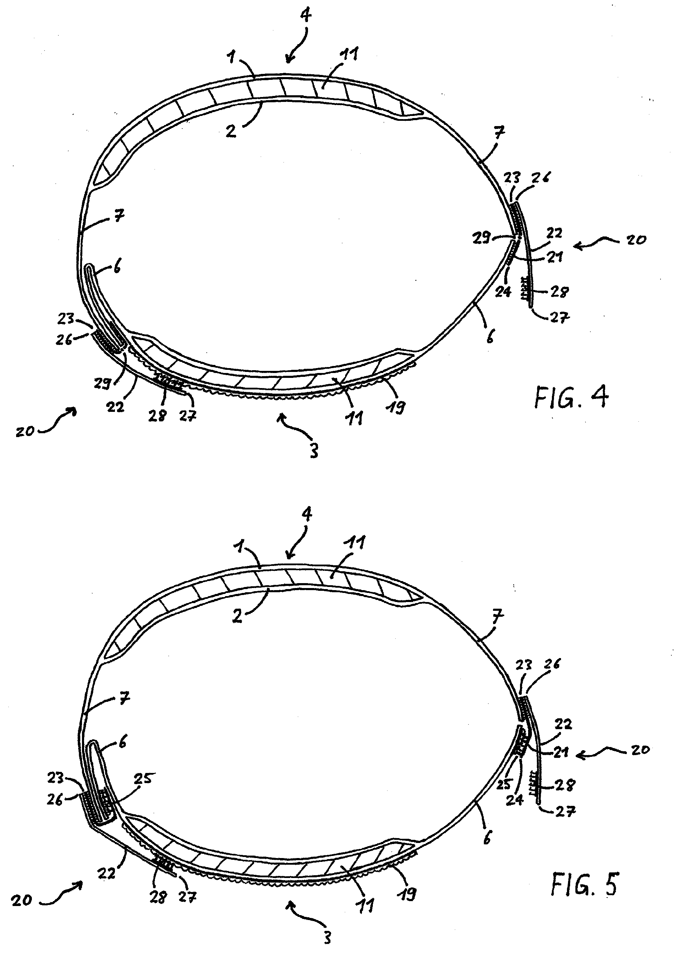

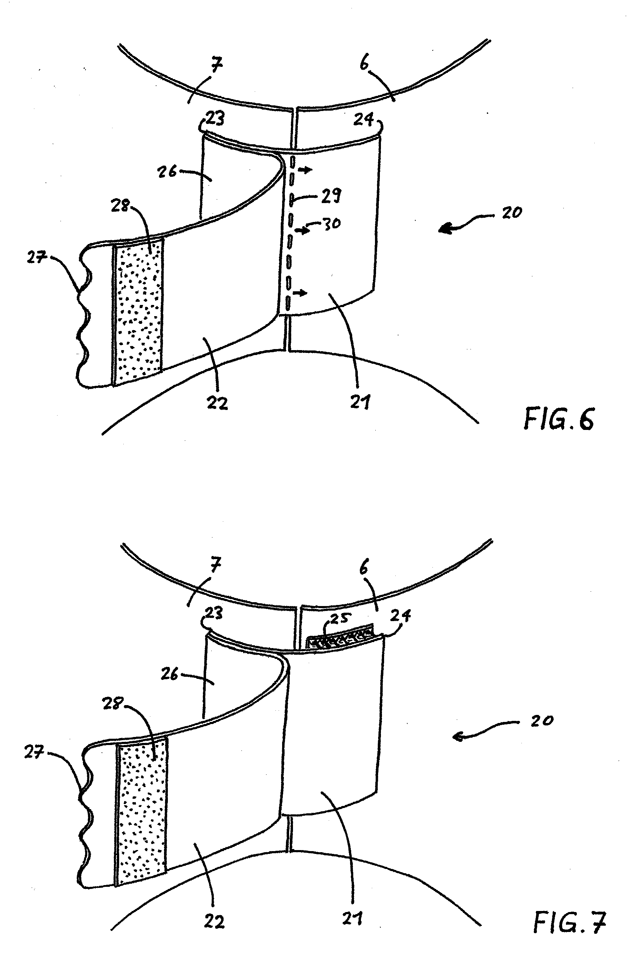

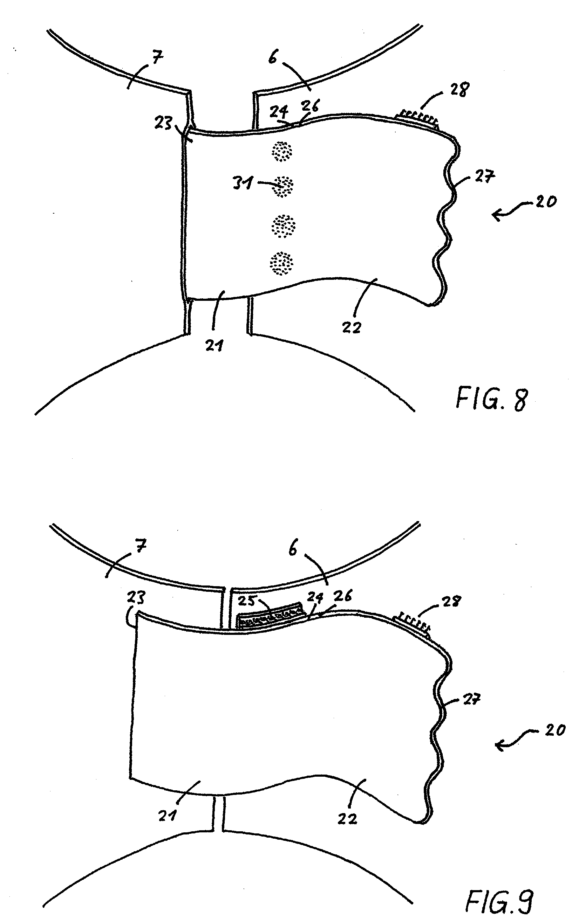

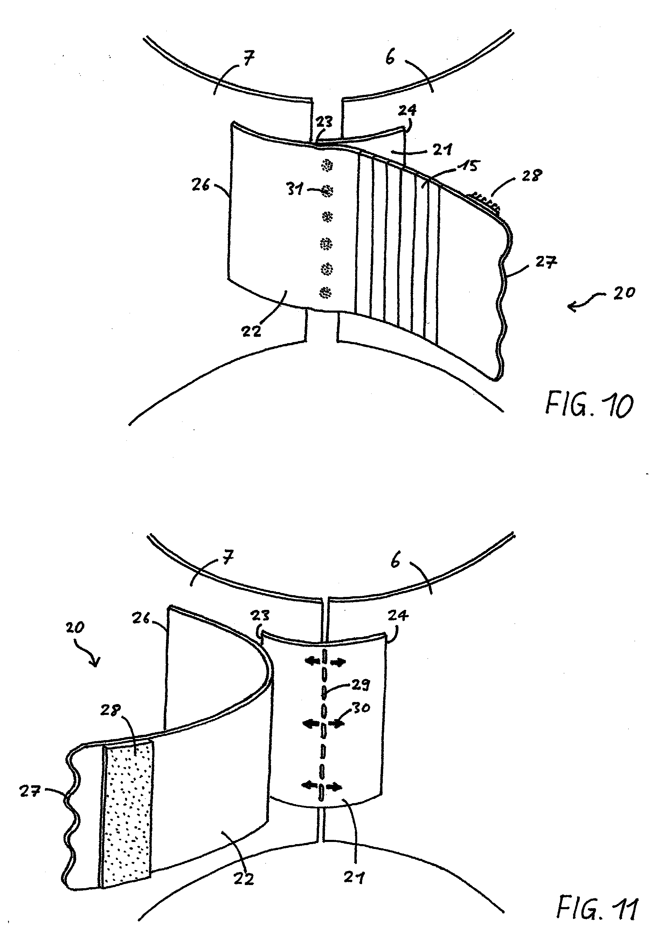

The primary fastener portion **21** connects the opposing front and back side parts **6, 7** along the longitudinal side edges of the front and back side parts. In a preferred embodiment shown in FIGS.**4** and **6,** a first end **23** of the primary fastener portion is permanently attached to the outer surface of the back side part **7** by an adhesive, thermal or ultrasonic bonding. Alter- natively, said first end **23** of the primary fastener portion may be attached on the inner surface or both, the outer and inner surface or between the layers of the back side panel. The second end **24** of the primary fastener portion is permanently attached on the outer surface of the front side part **6** by an adhesive, thermal or ultrasonic bonding. Alternatively, said second end **24** is releasable and refastenable attached on the outer surface of the front side part **6** by an adhesive or mechanical fastener **25,** such as hook and loop type fastener as shown in FIGS.**5** and **7.** Such, the opposing front and back side parts **6, 7** are connected only by the primary fastener portion **21** at the outermost longitudinal side edge what advantageously saves the welding of the overlapping or facing side parts and the cutting of the side part edges outside of the welding. Further, the application of such prefastening tapes **20** advantageously simplifies the manufacturing process and reduces raw material requirements, resulting in reduced manufacturing costs. Besides, the savings of raw material may be increased by a higher distance between the side edges of the opposing side parts as representatively shown in FIG.**8**. Still further, such prefastening tapes advantageously provides large leg openings and a large waist opening what allows the wearer or caregiver to insert legs and to pull up the diaper easily and without need of stretching the openings. All these advantages are maintained by the various embodiments of FIGS.**6** to **11.** FIGS.**6** and **11** representatively show fastener tapes **20** according to the present invention wherein both ends of the primary fastener portions **21** are perma- nently attached to the front and back side parts **6, 7.** To unfasten the prefastened diaper before or during use, the primary fastener portions 21 are manually tearable along a line of weakening **29** positioned between and parallel to the longitudinal side edges of the front and back side parts. The line of weakening **29** is preferably formed by point-wise cuts, perforation, thermal embossing or welding. Further, a primary fastener portion comprising a plurality of layers may provide the line of weakening by the interruption of on e of these layers. For convenient reasons, the line of weakening may be visibly marked by printed perforation patterns or printed diagrams **30** illustrating the tearable property of said line of weakening **29.** The unfastened diaper advantageously can be readily applied or removed like a conventional diaper. FIGS.**7** and **9** representatively show fastener tapes **20** according to the present invention in alternativ embodiments wherein the second end **24** of the primary fastener portion is releasable attached to the front side part **6** by a mechanical hook and loop type fastener member **25.** To unfasten the prefastened diaper before or during use, the mechanical fastener member **25** can be released from the front side part **6.** These embodiments advantageously allow to refasten the unfastened diaper and to restore the pant-like configuration. FIGS.**8** and **9** show preferred embodiments wherein the primary **21** and the secondary **22** fasteners are adjacent portions of one fastener strip. In FIG.**9**, the first end **23** of the primary fastener portion is permanently attached to the outer surface of the back side part **7** by an adhesive, thermal or ultrasonic bon- ding. Alternatively, said first end **23** of the primary fastener portion may be attached on the inner surface or both, the outer and inner surface or between the layers of the back side panel as representatively shown in FIG.**8**. The second end **24** of the primary fastener portion continuously proceed to the attached end **26** of the secondary fastener portion. The second end **24** comprises a manually tearable bonding section **31** and is releasably attached to the outer surface of the front side part **6** by an adhesive, thermal or ultrasonic bonding or by refastenable hook and loop type fastener members. To unfasten the prefastened diaper of these embodiments before or during use, the wearer or caregiver can laterally pull at the free end **27** of the secondary fastener portion **22** what applies peeling forces to the bonding section **31** and what results in peeling apart of the bonding. FIG.**10** shows a variation, wherein the primary fastener portion **21** is permanently attached to the front side part **6** and manually releasable attached to the secondary fastener portion **22** by an adhesive, thermal or ultrasonic bonding **31.** Such, the opposing front and back side parts **6, 7** are connected at the outermost longitudinal side edge by the second end **24** of the primary fastener portion and the attached end **26** of the secondary fastener portion laterally extending the first end of the primary fastener portion. Further, the embodiment of FIG.**10** may be performed by a primary fastener portion with a line of weakening according to the description above adjacent to an adhesive, thermal or ultrasonic bonding seam permanently attaching the primary fastener portion to the secondary fastener portion.

The secondary fastener portion **22,** as shown in a preferred embodiment in FIGS.**6** and **7,** comprises an attached end **26** and a free end **27** wherein the attached end is permanently attached to the outer surface of the first end **23** of the primary fastener portion by an adhesive, thermal or ultrasonic bonding. The free end **27** of the secondary fastener portion comprises a releasable and refastenable fastening member **28** such as adhesiv or mechanical hook type fastener and is in the ready to pull up configuration releasably and refastenably engaged to the outer surface of the front side part **6,** laterally extending the second end **24** of the primary fastener portion. The secondary fastener portion **22** provides in this ready to pull up configuration a high waist size diameter and prevents from unintentionally tearing the line of weakening **29** or the bonding section **31** of the primary fastener portion **21.** After pulling up the diaper over the hips of the wearer, the secondary fastener portion **22** is used like a conventional fastening tape on a conventional diaper what is well known to those skilled in the art. The wearer or caregiver adjusts and releasably attaches the secondary fastener portion on the outer surface of the front waist portion 3 or on an attachment panel **19** on said front waist portion, providing a conforming fit about the wearer's waist. FIG.**2** shows, that the side parts **6, 7** are folded and nestled against the wearer's hips by this adjustment step what advantageously reduces the diameter of the leg openings and thereby the risk of leakage. Further, the adjustment of the secondary fastener portion **22** advantageously keep the line of weakening **29** or the bonding section **31** of any forces during wearing the diaper. The secondary fastener portion is likewise used like a conventional fastening tape on a conventional diaper after unfastening the diaper before or during use. All these advantages are maintained by the various embodiments of FIGS.**6** to **11.** FIGS.**8** and **9** show another preferred embodiment of the secondary fastener portion, wherein the primary **21** and the secondary **22** fasteners are adjacent portions of one fastener strip. The second end **24** of the primary fastener portion continuously proceed to the attached end **26** of the secondary fastener portion. In the still further embodiment of FIG.**10** the attached end **26** laterally extends the first end **23** of the primary fastener portion and is permanently attached to the back side part **7.** The first end **23** of the primary fastener portion is releasably attached to the secondary fastener portion in a adhesive, thermal or ultrasonic bonding section **31.** Thus, the attached end **26** performs a part of the prefastening function of the primary fastener portion. In the still further embodiment of FIG.**11** the primary fastener **21** and the secondary fastener **22** are two not connected and adjacently attached tapes. Although it is typically preferred to close and adjust the diaper on the front side, it is understood that all embodiments of the fastener tape of the present invention could be attached vice versa on the front side parts and adjusted on the back side.

In a second aspect, the present invention concerns a fastener tape **20** for adjustable and pant-like prefastened diapers according to the description above. Several embodiments of fastener tapes for conventional diapers are well known to those skilled in the art. For example, so-called release tapes or Y-bond tapes are commercially available from Koester, Altendorf, Germany under the trade designation Precut CP-2/EM67. Such a fastener tape differs only in the lack of a line of weakening and in the method of application from a preferred prefastening tape according to the present invention as shown in FIG.**6.** The prefastening tape **20** of FIG.**6** comprises a primary fastener strip **21** made by a plastic film or a nonwoven material and performed with a manually tearable line of weakening **29** formed by point-wise cuts, perforation, thermal embossing or welding. For convenient reasons, the line of weakening **29** may be visibly marked by printed perforation patterns or printed diagrams **30** illustrating the tearable property of said line of weakening. The secondary fastener strip **22** comprises an attached end **26** and a free **27** end wherein the attached end **26** is permanently attached to the first end **23** of the primary fastener strip. The secondary fastener strip **22** is preferably made of a nonwoven material or a plastic film, formed as a single piece, although a plurality of pieces laterally in series, or a plurality of layers in series or partially or fully over- lapping and at least one of which is elastic may be used to form this strip. FIG.10 representatively shows a secondary fastener strip in a preferred embodiment with an elastic element 15 between the bonding section 31 and the fastening member 28. In each described embodiment, the free end 27 of the secondary fastener portion laterally extends the second end 24 of the primary fastener portion and comprises a releasable and refastenable hook type fastening member **28** attached to the inner surface of the secondary fastener portion by adhesive, thermal or ultrasonic bonding. Such fastening members are well known to those skilled in the art and comprise a plurality of hook elements made of nylon or any other suitable plastics. Each fastening region with which the respective fastening members are engaged comprises a bulky nonwoven fabric made of crimped fibers and corresponds to a plurality of loop elements. In FIGS.1 and 2 an attachment panel 19 on the front waist portion 3 of the diaper provides the loop type fastening region. Alternatively, two smaller laterally opposing attachment panels could be attached on the front waist portion. Furthermore, the nonwoven backsheet 1 itself could be bulky enough to provide loop fastener properties. FIGS.7 and 9 representatively show alternative embodiments of an adjustable and pant-like prefastening fastening tape according to the present invention and the description above. The second end 24 of the primary fastener is releasable and refastenable engaged to the front side part **6** by a hook and loop type fastener member **25,** thereby no tearable line of weakening is needed to unfasten the diaper. In the embodiment of FIG.**10**, the attached end **26** of the secondary fastener portion laterally extends the first end **23** of the primary fastener portion. The first end of the primary fastener portion is manually releasably attached to the secondary fastener portion by an adhesive, thermal or ultrasonic bonding section 31. Alternatively, the first end of the primary fastener portion is permanently attached to the secondary fastener portion and a line of weakening according to the description above allows to unfasten the primary fastener portion. In the embodiments of FIG.**8** and **9** the primary fastener **21** and the secondary fastener **22** are adjacent portions of one fastener strip. The fastener strip is preferably made of a nonwoven material or a plastic film, formed as a single piece in the embodiment shown in FIGS.**8** and **9,** although a plurality of pieces laterally in series, or a plurality of layers in series or partially or fully overlapping and at least one of which is elastic may be used to form this strip.

In a third aspect, the present invention concerns a method of producing a pant-like prefastened diaper comprising representatively the preferred steps of
a) providing a continuous web of interconnected absorbent chassis
b) attaching a pair of laterally opposed tape fasteners to the back or front side parts
c) selectively cutting said continuous web of interconnected absorbent chassis into discrete diapers
d) folding each of the discrete diapers about a fold line extending in a lateral direction through the crotch portion thereby positioning the front waist portion to the back waist portion and the front side parts to the back side parts in a facing relationship
e) prefastening the diaper by folding the laterally outward extending portions of the laterally opposed tape fasteners along the longitudinal side edges of the facing front and back side parts and attaching the folded fastener portion to the outer surface of the front or back side part respectively.

This method of producing advantageously provides a prefastening step similar to the proved attachment of conventional tape fasteners on conventional diapers.

## Claims

1. A prefastened disposable absorbent article which includes a front waist portion, a back waist portion, a crotch portion which extends between and connects said waist portions and two pairs of opposing front and back side parts wherein said absorbent article is pant-like prefastened by a pair of adjustable tape fastener comprising
a) a primary fastener portion which connects said opposing front and back side parts along the longitudinal side edges of said front and back side parts
b) a secondary fastener portion comprising an attached end and a free end wherein the attached end is permanently connected or attached to the primary fastener portion or to one of said opposing side parts and the free end is releasably and refastenably fastened to the other of said opposing side parts allowing to adjust and provide a conforming fit about a wearer's waist.

2. The prefastened absorbent article of claim **1** wherein at least one of said primary fastener portions is manually tearable or releasable along a longitudinal line of weakening to separate said opposing front and back side parts.

3. The prefastened absorbent article of claim **1** and **2** wherein said line of weakening is formed by point-wise cuts, perforation, adhesive seams or thermal or ultrasonic welding.

4. The prefastened absorbent article of claim **1** and **2** wherein the line of weakening is visibly marked by printed perforation patterns or printed diagrams illustrating the tearable or releasable property of said line of weakening.

5. The prefastened absorbent article of claim **1** wherein the primary fastener portion is permanently attached to both of said side parts by an adhesive coating or a thermal or ultrasonic welding.

6. The prefastened absorbent article of claim **1** wherein the primary fastener portion is permanently attached to one of said side parts and releasably attached to the other of said side parts by an adhesive coating or a thermal or ultrasonic bonding.

7. The prefastened absorbent article of claim **1** wherein the primary fastener portion is permanently attached to one of said side parts by an adhesive coating or a thermal or ultrasonic bonding and releasably and refastenably attached on the other of said side parts by an adhesive coating or a hook and loop type fastener.

8. The prefastened absorbent article of claim **1** wherein the primary fastener portion is permanently attached to one of said side parts and permanently or releasably attached to the secondary fastener portion by an adhesive coating, a thermal or ultrasonic bonding or a hook and loop type fastener.

9. The prefastened absorbent article of claim **1** wherein the attached end of the secondary fastener portion is permanently attached or laminated to the primary fastener portion or one of said side parts by an adhesive coating or a thermal or ultrasonic bonding and the free end of the secondary fastener portion is releasably and refastenably fastened to the other side part of the absorbent article by an adhesive coating or a hook and loop type fastener.

10. The prefastened absorbent article of claim **1** wherein the primary fastener and the secondary fastener are continuous portions of the same fastener strip and wherein said primary fastener portion is permanently attached to one of said side parts and releasably attached to the other of said side parts by an adhesive coating or a thermal or ultrasonic bonding and wherein said free end of the secondary fastener portion is releasably and refastenably attached to said other side part by an adhesive coating or a hook and loop type fastener.

11. The prefastened absorbent article of claim **1** wherein at least one of said secondary fastener portions includes an elastic or extensible member between said attached end and said free end providing a stretchable waist fit.

12. The prefastened absorbent article of claim **1** wherein at least one of said front and back side parts includes an elastic member providing a stretchable waist fit.

13. The prefastened absorbent article of claim **1** wherein at least one of said front and back waist portions includes an elastic member providing a stretchable waist fit.

14. A fastener tape for adjustable and pant-like prefastened absorbent articles according to any one of the preceding claims comprising
a) a primary fastener portion with a manually releasable bonding section or a manually tearable line of weakening
b) a secondary fastener portion with an attached end and a free end wherein the attached end is permanently connected, attached or laminated to the primary fastener portion and the free end laterally extends the primary fastener portion and comprises a releasable and refastenable adhesive coating or a hook type fastener.

15. The fastener tape of claim **14** wherein said line of weakening is formed by point-wise cuts, perforation, adhesive seams, thermal or ultrasonic welding.

16. The fastener tape of claim **14** wherein said line of weakening is visibly marked by printed perforation patterns or printed diagrams illustrating the tearable or releasable property of said line of weakening.

17. The fastener tape of claim **14** wherein said manually releasable bonding section comprises a refastenable adhesive coating or a refastenable hook and loop type fastener.

18. The fastener tape of claim **14** wherein said attached end of the secondary fastener portion laterally extends the primary fastener portion.

19. The fastener tape of claim **14** wherein the primary fastener and the secondary fastener are continuous portions of the same fastener strip.

20. The fastener tape of claim **14** wherein the secondary fastener portion includes an elastic or extensible member between said attached end and said free end.

21. A method of producing a prefastened disposable absorbent article of claim **1** comprising the steps of
a) providing a continuous web of interconnected absorbent chassis
b) attaching a pair of laterally opposed tape fasteners to the back or front side parts
c) selectively cutting said continuous web of interconnected absorbent chassis into discrete absorbent articles
d) folding each of said discrete absorbent articles about a fold line extending in a lateral direction through said crotch portion thereby positioning said front waist portion to said back waist portion and said front side parts to said back side parts in a facing relationship
e) prefastening said discrete absorbent articles by folding the outstanding portions of said laterally opposed tape fasteners along the longitudinal side edges of said facing front and back side parts and attaching said folded fastener portion to the outer surface of the front or back side parts respectively.

22. The method of producing a prefastened disposable absorbent article of claim **21** in the various embodiments of claim **1** to **13.**
